# EUROPEAN PATENT APPLICATION

(11) **EP 2 667 180 A1**
(43) Date of publication of application: **27.11.2013**
(21) Application number: 12737158.1
(22) Date of filing: 19.01.2012
(51) Int. Cl.: G01N 21/01, G01N 21/51, G01N 35/04

(54) **AUTOMATIC ANALYSIS DEVICE**

(30) Priority: 21.01.2011 JP 2011010349
(71) Applicant: Hitachi High-Technologies Corporation, Tokyo 105-8717 (JP)
(72) Inventor: WAKUI Akihito, Hitachinaka-shi Ibaraki 312-8504 (JP); YAMAZAKI Hajime, Hitachinaka-shi Ibaraki 312-8504 (JP)
(74) Representative: Beetz & Partner
(86) International application number: PCT/JP2012/051117
(87) International publication number: WO 2012/099215

(57) **Abstract**

The invention provides an automatic analyzer having a scattered-light detecting optical system in which a light source is disposed at an angle with respect to a plane of a reaction vessel. This reduces the amount of a sample-reagent mix required for an analysis, thereby reducing the running cost of reagents as well, and also allows relatively free design of reaction vessels in terms of their sizes and shapes. The automatic analyzer includes: a reaction vessel in which a sample is caused to react with a reagent; a reaction disk on which to place reaction vessels in the form of a circle; a reaction disk rotating mechanism for rotating the reaction disk; a light source for radiating light to be measured onto one of the reaction vessels; and a photodetector for detecting transmissive light radiated from the light source and passing through a sample-reagent mix in the one of the reaction vessels. The automatic analyzer further includes an optical system for causing the light source to radiate light onto one of the reaction vessels at an angle with respect to a plane of the one of the reaction vessels.

## Description

### Technical Field

The present invention relates to automatic analyzers that examine substances in biological samples such as blood and urine and particularly to an automatic analyzer having a scattered-light measuring device.

### Background Art

An example of an automatic analyzer used for clinical purposes is a biochemical analyzer that examines a particular substance in a biological sample (e.g., blood and urine) by using a reagent that exhibits color changes when reacting with that substance. Such an analyzer is designed to radiate light onto a sample-reagent mix and measure the intensity of transmissive light passing through the sample-reagent mix on a wavelength-by-wavelength basis, thereby measuring color changes quantitatively.

Other than such analyzers that examine transmissive light, there are also medical analyzers that measure the size or amount of particles in a sample using scattered light. Such analyzers detect, for example, solid substances floating in urine or perform an immunoassay by examining agglutination reactions of latex particles. Such analyzing methods are disclosed in Patent Documents 1 and 2 shown below, for example.

### Prior Art Document

### Patent Documents

Patent Document 1: JP-2010-32505-A
Patent Document 2: JP-2007-309765-A

### Summary of the Invention

### Problems to be Solved by the Invention

To measure scattered light, a photodetector is disposed at an angle with respect to the axis of light radiated from a light source. However, when the light scattered from a sample-reagent mix in a reaction vessel is measured, the intensity of the scattered light varies if it passes through the surface of the sample-reagent mix or through a corner of the reaction vessel. To ensure consistent measurements, it is therefore desired to measure only the scattered light passing through the same plane of the reaction vessel. However, the installation position of the photodetector within an automatic analyzer is limited because reaction vessels used are small and also because the analyzer often includes a thermostat tank for maintaining the temperatures of the reaction vessels at a constant value.

An object of the invention is to provide an automatic analyzer having a scattered-light detecting optical system that allows relatively free design of reaction vessels in terms of their sizes and shapes.

### Means for Solving the Problems

To achieve the above object, the invention provides an automatic analyzer configured as follows.

The automatic analyzer includes: a reaction vessel in which a sample is caused to react with a reagent; a reaction disk on which to place reaction vessels in the form of a circle; a reaction disk rotating mechanism for rotating the reaction disk; a light source for radiating light to be measured onto one of the reaction vessels; and a photodetector for detecting transmissive light radiated from the light source and passing through a sample-reagent mix in the one of the reaction vessels. The automatic analyzer further includes an optical system for causing the light source to radiate light onto one of the reaction vessels at an angle with respect to a plane of the one of the reaction vessels.

The above description may also be represented as follows.

The automatic analyzer includes: a reaction vessel in which a sample is caused to react with a reagent; a reaction disk on which to place reaction vessels in the form of a circle; a reaction disk rotating mechanism for rotating the reaction disk; a light source for radiating light to be measured onto one of the reaction vessels; and a photodetector for detecting transmissive light radiated from the light source and passing through a sample-reagent mix in the one of the reaction vessels. The reaction vessels are cuboid-shaped or cylinder-shaped, and the automatic analyzer further includes an optical system for causing the light source to radiate light onto one of the reaction vessels at an angle with respect to a plane of the one of the reaction vessels.

In a conventional optical system for scattered light detection, a light source is often disposed on an axis that passes through the center of a reaction vessel and is parallel to a longitude line of a reaction disk, so that measurement of transmissive light can also be performed. In other words, when the reaction vessel is cuboid-shaped, the common practice is to cause the light source to radiate light onto the reaction vessel at 90 degrees with respect to a surface of the reaction vessel. A transmissive-light detector is also disposed on the same axis. Under this method, the angle of scattered light relative to the optical axis of the light source cannot be increased too much. This is because, when the angle of scattered light is increased excessively relative to a horizontal plane, the scattered light may collide with the boundary between the reaction vessel and the surface of a sample-reagent mix within the vessel or with a bottom corner of the vessel. In contrast, the present invention is characterized in that the optical axis of the radiated light is disposed at a particular angle so that the angle of scattered light relative to a horizontal or vertical plane can be made larger.

### Effect of the Invention

In accordance with the invention, it is possible to provide an automatic analyzer having a scattered-light detecting optical system that allows relatively free design of reaction vessels in terms of their sizes and shapes.

### Brief Description of the Drawings

FIG. 1 illustrates how to detect scattered light;
FIG. 2 illustrates a positional relationship between a light source and a photodetector according to a conventional method of scattered light detection;
FIG. 3 illustrates a positional relationship between a light source and a photodetector according to an embodiment of the present invention in which both the light source and the photodetector are disposed at angles with respect to a horizontal plane;
FIG. 4 illustrates how to reduce the amount of a sample-reagent mix according to the embodiment of the invention;
FIG. 5 illustrates a positional relationship between a light source and a photodetector according to another embodiment of the invention in which both the light source and the photodetector are disposed at angles with respect to a vertical plane; and
FIG. 6 illustrates an entire configuration of an automatic analyzer according to a further embodiment of the invention.

### Mode for Carrying out the Invention

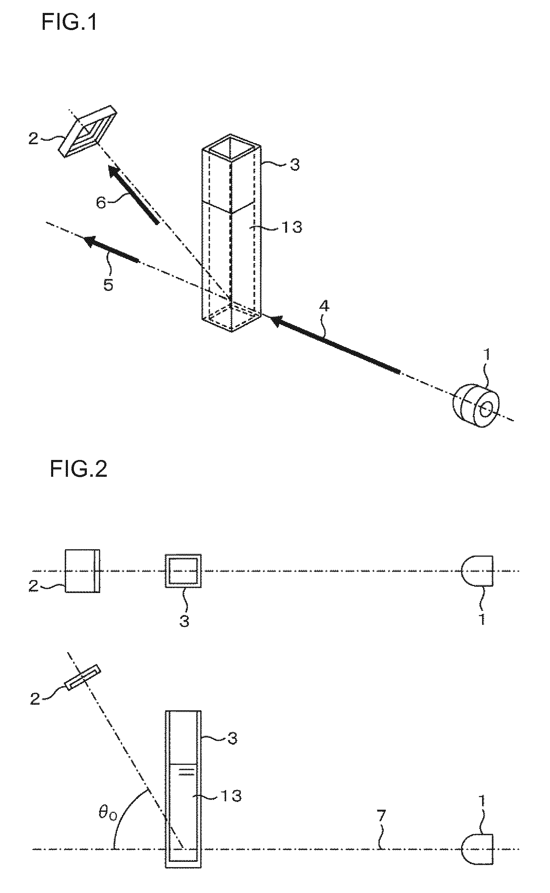

Embodiments of the present invention will now be described with reference to FIGS. 1 to 6.

FIG. 1 illustrates how to detect scattered light. When a light source 1 radiates light 4 onto a reaction vessel 3, the light 4 is separated into transmissive light 5 and scattered light 6. A photodetector 2 is used to detect this scattered light. Note that while the following explanation is based on an assumption that the reaction vessel 3 is cuboid-shaped, it can instead be shaped into a cylinder or the like.

FIG. 2 illustrates a positional relationship between a light source and a photodetector according to a conventional method of scattered light detection. The light source 1, the photodetector 2, and the reaction vessel 3 are now arranged on the same straight line. Also, the photodetector 2 is disposed at an angle θ₀ with respect to a horizontal plane 7.

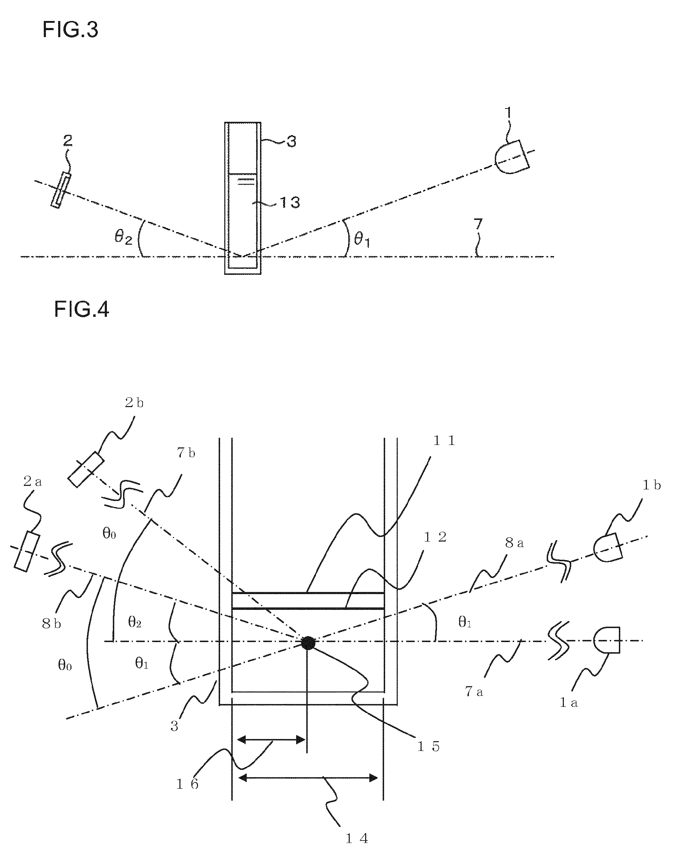

FIG. 3 illustrates a positional relationship between the light source 1 and the photodetector 2 according to an embodiment of the present invention in which both the light source 1 and the photodetector 2 are disposed at angles with respect to the horizontal plane. The light source 1 and the photodetector 2 are disposed at angles θ₁ and θ₂, respectively, relative to the horizontal plane 7.

FIG. 4 illustrates how to reduce the amount of a sample-reagent mix according to the embodiment of the invention. In this figure, reference symbol 1a represents a light source according to a conventional method while 1b represents a light source according to the present invention. Also, reference symbol 2b represents a photodetector according to a conventional method while 2a represents a photodetector according to the present invention. FIG. 4 further shows an axis 7b disposed at an angle θ₀ with respect to a horizontal plane, an axis 8a disposed at an angle θ₁ with respect to the plane, and an axis 8b disposed at an angle θ₂ with respect to the plane. The axis 7b represents a scattered-light detecting axis for the photodetector according to a conventional method while the axis 8b represents a scattered-light detecting axis for the photodetector according to the present invention. The axis 8a is an optical axis of the light source according to the present invention.

When the angle θ₀ is assumed to be the optimal angle for scattered light detection, the light source angle θ₁ and the detection angle θ₂ are determined such that θ₁ + θ₂ = θ₀. Because the height of the surface of the sample-reagent mix is smallest when the optical axis θ₁ of the radiated light is equal to the angle θ₂ set for the scattered-light detector (when θ₁ = θ₂), the angle θ₂ set for the scattered-light detector is equal to θ₀/2.

Assume, for example, that θ₀ = 45°, the reaction vessel is square in cross section, and the width 14 of the reaction vessel 3 is 5 mm. Further assume that the optical axis 8a of the radiated light and the scattered-light detecting axes 8b and 7b cross at a point 15 and that the distance 14 inside the reaction vessel (the width of the reaction vessel) is 2.5 mm. In that case, the liquid surface height can be in the range of 1 mm (2.5 mm × tan 22.5°) to 2.5 mm (2.5 mm × tan 45°).

Because a cross section of the reaction vessel is 25 mm² (5 mm × 5 mm), the amount of the sample-reagent mix can be reduced to the range of 25 µl to 62.5 µl. Accordingly, the running cost of reagents can be reduced approximately by half.

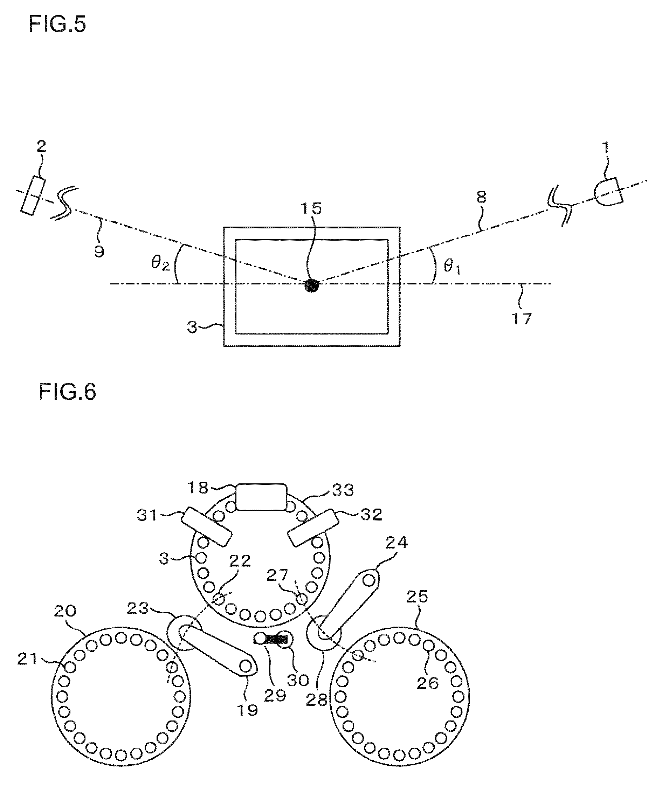

FIG. 5 illustrates a positional relationship between the light source 1 and the photodetector 2 according to another embodiment of the invention in which both the light source 1 and the photodetector 2 are disposed at angles with respect to a vertical plane 17. The light source 1 and the photodetector 2 are disposed at angles θ₁ and θ₂, respectively, relative to the vertical plane 17. When the light source 1 and the photodetector 2 are disposed at angles with respect to the vertical plane 17, not to a horizontal plane, they can be installed at lower positions, thus reducing the overall height of the analyzer. This arrangement, however, places an upper limit on measurement time if the analyzer is designed to measure transmissive light through or scattered light from a reaction vessel during the rotation of its reaction disk. The choice of reaction vessel sizes is also limited when smaller amount of samples and reagents need to be used.

FIG. 6 illustrates an automatic analyzer to which the invention is applied.

A reaction vessel rinse mechanism 18 cleans reaction vessels by discharging and suctioning water or a detergent into and from the reaction vessels 3. A sample dispenser 19 suctions a sample from one of sample vessels 21 placed on a sample vessel setting table 20, transfers the sample to a sample dispensing position 22 of a reaction disk, and discharges the sample into one of the reaction vessels 3. After the sample discharge, the sample dispenser 19 is cleaned by a sample dispenser rinse mechanism 23. A reagent dispenser 24 suctions a reagent from one of reagent vessels 26 placed on a reagent vessel setting table 25, transfers the reagent to a reagent dispensing position 27 of the reaction disk, and discharges the reagent into the sample-containing reaction vessel 3. After the reagent discharge, the reagent dispenser 24 is cleaned by a reagent dispenser rinse mechanism 28. The sample to be examined and the reagent are mixed by a stirring mechanism 29, which is cleaned by a stirring mechanism rinse mechanism 30 after the mixing. A transmissive-light measuring unit 31 measures the absorbance of the sample-reagent mix contained within the reaction vessel 3 while a scattered-light measuring unit 32 measures scattered light generated from the sample-reagent mix contained within the reaction vessel 3. All of the above operations including the measurement of transmissive light and scattered light are performed while the reaction disk 33 is being operated.

### Description of Reference Numerals

1: Light source
1a: Light source before the present invention is executed
1b: Light source after the present invention is executed
2: Detector
2a: Detector after the present invention is executed
2b: Detector before the present invention is executed
3: Reaction vessel
4: Incident light
5: Transmissive light
6: Scattered light
7: Horizontal plane
7a: Horizontal plane
7b: Axis disposed at an angle θ₀ with respect to a horizontal plane
8: Optical axis of a light source after the present invention is executed
8a: Axis disposed at an angle θ₁ with respect to a horizontal plane
8b: Axis disposed at an angle θ₂ with respect to a horizontal plane
11: Liquid surface height before the present invention is executed
12: Liquid surface height after the present invention is executed
13: Sample-reagent mix (latex particles included)
14: Width of a reaction vessel
15: Intersecting point between an optical axis and light detecting axes
16: Distance between an inner wall of a reaction vessel and the intersecting point 15 between an optical axis and light detecting axes
17: Vertical plane
18: Reaction vessel rinse mechanism
19: Sample dispenser
20: Sample vessel setting table
21: Sample vessel
22: Sample dispensing position on a reaction disk
23: Sample dispenser rinse mechanism
24: Reagent dispenser
25: Reagent vessel setting table
26: Reagent vessel
27: Reagent dispensing position on a reaction disk
28: Reagent dispenser rinse mechanism
29: Stirring mechanism
30: Stirring mechanism rinse mechanism
31: Transmissive-light measuring unit
32: Scattered-light measuring unit
33: Reaction disk

## Claims

1. An automatic analyzer comprising:
a reaction vessel in which a sample is caused to react with a reagent;
a reaction disk on which to place reaction vessels in the form of a circle;
a reaction disk rotating mechanism for rotating the reaction disk;
a light source for radiating light to be measured onto one of the reaction vessels; and
a photodetector for detecting transmissive light radiated from the light source and passing through a sample-reagent mix in the one of the reaction vessels,
wherein the automatic analyzer further includes an optical system for causing the light source to radiate light onto one of the reaction vessels at an angle with respect to a plane of the one of the reaction vessels.

2. An automatic analyzer comprising:
a reaction vessel in which a sample is caused to react with a reagent;
a reaction disk on which to place reaction vessels in the form of a circle;
a reaction disk rotating mechanism for rotating the reaction disk;
a light source for radiating light to be measured onto one of the reaction vessels; and
a photodetector for detecting transmissive light radiated from the light source and passing through a sample-reagent mix in the one of the reaction vessels,
wherein the reaction vessels are cuboid-shaped or cylinder-shaped, and
the automatic analyzer further includes an optical system for causing the light source to radiate light onto one of the reaction vessels at an angle with respect to a plane of the one of the reaction vessels.

3. The automatic analyzer according to claim 1 or 2, wherein the photodetector is disposed at an angle with respect to an axis of the light radiated from the light source onto the one of the reaction vessels.

4. The automatic analyzer according to claim 3, wherein an angle of the light radiated from the light source onto the one of the reaction vessels is equal to an angle between the photodetector and the one of the reaction vessels.
